# EUROPEAN PATENT SPECIFICATION

(11) **EP 1 843 736 B1**
(45) Date of publication and mention of the grant of the patent: **08.09.2010**
(21) Application number: 05850341.8
(22) Date of filing: 20.12.2005
(51) Int. Cl.: A61K 8/00, A61K 8/04, A61K 8/26, A61K 8/41, A61K 8/89, A61Q 15/00, B01F 3/08

(54) **METHOD OF PREPARING CARRIER LIQUIDS**
VERFAHREN ZUR HERSTELLUNG VON TRÄGERFLÜSSIGKEITEN
PROCEDE DE PREPARATION DE LIQUIDES SUPPORTS

(30) Priority: 28.01.2005 GB 0501833
(43) Date of publication of application: 17.10.2007
(73) Proprietor: Unilever PLC, London EC4Y 0DY (GB); Unilever N.V., 3013 AL Rotterdam (NL)
(72) Inventor: COOPER, Andrew Ian University of Liverpool, Liverpool Merseyside CH69 3BX (GB); DUNCALF, David, Wirral Merseyside CH63 3JW (GB); FOSTER, Alison Jayne, Wirral Merseyside CH6 3 3JW (GB); RANNARD, Stephen Paul, Wirral Merseyside CH 63 3JW (GB); WINTER, Jeremy Nicholas, Wirral Merseyside CH63 3JW (GB); ZHANG, Haifei University of Liverpool, Liverpool Merseyside CH69 3BX (GB)
(74) Representative: Elliott, Peter William
(86) International application number: PCT/EP2005/013935
(87) International publication number: WO 2006/079410

(56) References cited:
- EP-A- 1 072 199
- GB-A- 736 433
- US-A- 6 146 663

## Description

### Technical Field:

The present invention relates to carrier liquids and to methods of producing such liquids.

### Background to the Invention:

Our co-pending WO 2004/011 537 describes the formation of solid, porous beads comprising a three dimensional open-cell lattice of a water-soluble polymeric material with an average bead diameter in the range 0.2 to 5mm. These are typically 'templated' materials formed by the removal of a both the continuous and dispersed phase from a high internal phase emulsion. This leaves a 'skeletal' form of the emulsion behind. The beads dissolve rapidly in water and have the remarkable property that water insoluble components dispersed in the emulsion prior to drying can also be dispersed in water on solution of the beads.

There are many instances in personal care products such as deodorants, skin and hair cleaning or care products or in household products such as laundry cleaning and care products or household cleaning or care products for hard and soft surfaces where it is desirable to administer hydrophobic materials (for example perfume oils, sunscreens etc.) in an aqueous environment. Conversely there are cases in which a hydrophilic material (for example a certain water-soluble anti-perspirant actives) need be dispersed in a non-aqueous environment.

As an example to illustrate the problem, activated aluminium chlorohydrate (AACH) and diethylenetriaminepentaacetic acid (DTPA) are both active materials in anti-perspirant compositions and are water-soluble. It would be useful to be able to deliver both of these materials from a non-aqueous, silicone-based, composition.

### Brief Description of the Invention:

We have devised what are known herein as 'carrier liquids'. These are compositions which are liquid at ambient temperature (25 Celsius) and contain a phase-incompatible material in a disperse form. By `phase-incompatible' we mean that the material is not normally soluble in the liquid, i.e. a solid body of the material introduced into the liquid will remain as such without dissolving.

The present invention provides a method for the preparation of a carrier liquid which comprises the steps of:
(I) preparing an emulsion comprising:
   a) An aqueous phase,
   b) A second liquid phase which is volatile and which is immiscible with aqueous phase (a),
   c) A carrier material which is soluble in the continuous phase of the emulsion and which is liquid at ambient temperature, and
   d) A dopant which is soluble in the disperse phase of the emulsion,
(II) cooling the emulsion produced in step (I) to a temperature at which at least both the continuous phase and the carrier material become solid,
(III) removing water and the volatile second phase from the cooled emulsion in vapour form, and,
(IV) returning the product of step (III) to ambient temperature to obtain a liquid product with the dopant dispersed therein.

Due to the presence of the carrier material in the continuous phase, the cooled emulsion is believed to retain its structure when the volatile components of the phases are removed leaving a skeletal lattice of the carrier material. For this reason it may be described as a 'templated' material. It is believed that the phase-incompatible dopant material is finely dispersed therein, possibly at a nano-disperse or molecular scale.

On return to ambient temperature, the carrier material melts, forming a liquid. Surprisingly, the dispersed insoluble dopant material remains in a dispersed form through the liquid carrier material, although it would not normally be soluble in the carrier material. When the carrier liquid is added to further miscible liquid the dopant can readily be spread through the resulting mixture whether it is soluble in the mixture or not.

### Detailed Description of the Invention:

In order that the present invention may be better understood and carried forth into practice, it is described below with reference to various preferred features and particular embodiments. While the invention is particularly described with reference of non-aqueous compositions in the field of cosmetics, this in not intended to limit the scope of the invention.

### Carrier Materials:

The carrier material may either be water-soluble or soluble in some non-aqueous, water immiscible solvent. In all cases it will be present in the continuous phase of the emulsion of step (I).

Therefore, if the carrier material is water soluble it will be introduced through the aqueous phase and the aqueous phase will be the continuous phase of the emulsion made in step (I) .

Alternatively, and more preferably, if the carrier material is not soluble in water then it will be introduced through the second liquid phase and this second liquid phase will form the continuous phase of the emulsion made in step (I).

Examples of suitable water-insoluble carrier materials include natural oils such as triglycerides, mineral oils or synthetic oils. These are preferably those commonly used in cosmetic formulations. Preferred natural oils include vegetable oils, preferably avocado oil, rice bran oil, jojoba oil and Babassu oil. Preferred non-vegetable oils include silicone oils and paraffin oils. Mixtures of oils can be used.

Also amongst the preferred carrier materials are:
- linear or branched esters of fatty acids and alcohols (such as C12-C13 Alkyl Octanoate esters),
- esters of fatty acids and glycols (such as propylene glycol esters)
- esters of hydroxyfatty acids, including C12-C13 Alkyl Malate, C12-C13 Alkyl Lactate and C12-C13 Alkyl Citrate.

Other esters such as diethylene glycol dioctanoate or diisononanate, propylene glycol dicaprylate, neopentyl glycol diheptanoate etc. can be used.

The water-immiscible carrier material can contain lipophilic agents which are dissolved therein. These can be, for example:
- UV-blocking agent such as Octyl Methoxycinnamate, Octyl Salicylate; Homosalate; Menthyl Anthranilate; Octocrylene; Benzophenone-3; Octyl Dimethyl PABA [p-aminobenzoic acid]; 4-Methylbenzilidene Camphor; Butyl Methoxy-Dibenzoyl methane,
- liposoluble vitamins such as vitamin A esters (Retinol palmitate, acetate); vitamin E such as Tocopherol acetate or Tocopherol linolate; vitamin B2, vitamin D6 ; vitamin F;
- anti-inflammatory agents such as Bisabolol, Glycerrethinic acid, Stearyl Glycerrhetinate;
- polyunsaturated fatty acids or fatty acid esters thereof such as avocado, peanut and borrage oils; jojoba oil and calendula oil etc.;
- unsaponifiables such as shea butter, avocado, soybean oil etc.;
- lanolin and lanolin derivatives;
- emollients such as perhydrosqualene, perfluoropolyethers.

Silicone oil is preferred as a carrier material for cosmetic applications because it does not feel greasy, shows good spreadability on the skin and good water repellence. Suitably non-volatile silicone oils comprise dimethicones or linear silicone oils containing a high proportion of phenyl substituents. Preferred examples of non-volatile silicone oils which are suitable as carrier materials include members of the DC200 series and DC704 as available from Dow Corning. Linear or cyclic polydimethylsiloxanes such as Cyclomethicone to which further organopolysiloxanes can be added such as Alkyldimethicone, Alkoxydimethicone (Abil^{™} ex Goldschmidt), or Phenyldimethicone or Phenyltrimethicone can be used.

In the alternative the carrier material can be water-soluble. Preferred water-soluble carrier materials include surfactants, which are liquid at ambient temperatures. The surfactant may be non-ionic, anionic, cationic, amphoteric or zwitterionic.

Examples of suitable non-ionic surfactants include ethoxylated triglycerides; fatty alcohol ethoxylates; alkylphenol ethoxylates; fatty acid ethoxylates; fatty amide ethoxylates; fatty amine ethoxylates; sorbitan alkanoates; ethylated sorbitan alkanoates; alkyl ethoxylates; pluronics^{™}; alkyl polyglucosides; stearol ethoxylates; alkyl polyglycosides.

Examples of suitable anionic surfactants include alkylether sulfates; alkylether carboxylates; alkylbenzene sulfonates; alkylether phosphates; dialkyl sulfosuccinates; alkyl sulfonates; soaps; alkyl sulfates; alkyl carboxylates; alkyl phosphates; paraffin sulfonates; secondary n-alkane sulfonates; alpha-olefin sulfonates; isethionate sulfonates.

Examples of suitable cationic surfactants include fatty amine salts; fatty diamine salts; quaternary ammonium compounds; phosphonium surfactants; sulfonium surfactants; sulfonxonium surfactants.

Examples of suitable zwitterionic surfactants include N-alkyl derivatives of amino acids (such as glycine, betaine, aminopropionic acid); imidazoline surfactants; amine oxides; amidobetaines.

Mixtures of surfactants may be used and/or other hydrophilic carrier materials can be used.

### Second Phase:

The water-immiscible second liquid phase of the emulsion may be selected from one or more from the following group of volatile organic solvents:
   - alkanes, such as heptane, n-hexane, isooctane, dodecane, decane;
   - cyclic hydrocarbons, such as toluene, xylene, cyclohexane;
   - halogenated alkanes such as dichloromethane, dichoroethane, trichloromethane (chloroform), fluorotrichloromethane and tetrachloroethane;
   - esters such as ethyl acetate;
   - ketones such as 2-butanone;
   - ethers such as diethyl ether;
   - volatile cyclic silicones such as either linear or cyclomethicones containing from 4 to 6 silicon units. Suitable examples include DC245 and DC345, both of which are available from Dow Corning Inc.
and mixtures thereof

By 'volatile' here is meant that the second liquid phase is more volatile than the carrier material. This enables the second liquid phase to be removed as a vapour phase, such as by freeze-drying, without removing the carrier material.

Preferably, the second liquid phase comprises from about 10 % to about 95 % v/v of the emulsion, more preferably from about 20 % to about 60 % v/v.

A particularly preferred solvent is cyclohexane. The freezing point of cyclohexane is higher than that of water and the specific heat capacity for cyclohexane is much lower than that of water. It is believed that this assists rapid freezing of the emulsion.

### Dopants:

The dopant is phase-incompatible with the carrier material. Thus hydrophobic dopants have a water miscible carrier material and hydrophillic dopants have a carrier material that is not water miscible. As the carrier material is incorporated into the continuous phase of the emulsion, so the dopant is incorporated in the dispersed phase.

Thus, hydrophillic dopants may be incorporated in water-immiscible liquids by incorporating them in the dispersed water phase of a water-in-oil emulsion, which is then freeze-dried. In the alternative, hydrophobic dopants may be incorporated by dissolving them in the discontinuous oil phase of an oil-in-water emulsion, which is then freeze-dried.

The use of the carrier liquids of the present invention facilitates dispersion and in many cases enables materials to be dispersed more effectively than previously.

The carrier liquids of the present invention may be used to introduce phase-incompatible materials into products, during the manufacture of the products.

The carrier liquids of the present invention may be used to transport materials to sites where they can be incorporated into products.

Hydrophobic dopants may include the lipophilic agents mentioned above. In particular, hydrophobic dopants may include:-
- antimicrobial agents, for example: triclosan, climbazole, octapyrox, ketoconizole, phthalimoperoxyhexanoic acid (PAP), quaternary ammonium compounds, colloidal silver, zinc oxide.
- antidandruff agent for example: zinc pyrithione
- skin lightening agents for example 4-ethylresorcinol
- fluorescing agents for example: 2,5-bis(2-benzoxazolyl) thiophene for use on fabrics (such as cotton, nylon, polycotton or polyester)in laundry products
- skin conditioning agents, for example cholesterol
- antifoaming agents for example isoparrafin
- hair conditioning agents for example quaternary ammonium compounds, protein hydrolysates, peptides, ceramides and hydrophobic conditioning oils for example hydrocarbon oils such as paraffin oils and/or mineral oils, fatty esters such as mono-, di-, and triglycerides, silicone oils such as polydimethylsiloxanes (e.g. dimethicone) and mixtures thereof
- fabric conditioning agents for example quaternary ammonium compounds having 1 to 3, preferably 2 optionally substituted (C8-C24) alk(en)yl chains attached to the nitrogen atom by one or more ester groups; hydrophobic monoparticles such as a sucrose polyester for example sucrose tetra-tallowate; silicones for example polydimethylsiloxane
- thickening agents for example hydrophobically modified cellulose ethers such as modified hydroxyethylcelluloses
- dyes for example dyes intended to change the colour of fabrics, fibres, skin or hair.
- UV protecting agents such as sunscreens for example octyl methoxycinnamate (Parsol MCX), butyl methoxydibenzoylmethane (Parsol 1789) and benzophenone-3 (Uvinul M-40), ferulic acid.
- bleach or bleach precursors for example 6-N-phthalimidoperoxyhexanoic acid (PAP) or photobleaching compounds. Dispersing the bleach from carrier liquids of the present invention results in the bleach being more finely dispersed and reduces the spot damage seen when larger particles of the bleach contact a fabric
- antioxidants for example hydrophobic vitamins such as vitamin E, retinol, antioxiants based on hydroxytoluene such as Irganox^{™} or commercially available antioxidants such as the Trollox^{™} series.
- insecticides, pesticides, herbicides that are stored as solid compositions before use but which are made up into liquid for spraying onto animals or crops
- perfumes or flavourings or precursors thereto
- pharmaceutically or veterinary active materials.

Some specific examples of products in which the carrier liquids of the present invention may be used are given below. These are given as examples only and are not intended to limit the applicability of the present invention. Examples of situations where the carrier liquids of the present invention are used to incorporate a hydrophobic material into a product during the manufacture of that product include:-
- the introduction of hydrophobic materials such as fluorescers; enzymes; bleaches; hydrophobic polymers for example hydrophobically modified polyacrylates, silicones, hydrophobically modified polyvinylpyrrolidone, sulpha alkyl polysaccharides, Jaguar^{™} and JR polymers; fatty alcohols or acids; dyes for example shading dyes or black dyes for colour recovery into laundry products.
- the use of carrier liquids according to the present • invention containing hydrophobic dyes in the manufacture of water soluble inkjet compositions.
- the introduction of carrier liquids containing different hydrophobic materials enables a manufacturer to produce a single base formulation into which the desired hydrophobic materials may be introduced by the use of the appropriate carrier liquids of the present invention.

As well as the normally insoluble dopant, the carrier liquids of the present invention may also include materials that are soluble in the carrier liquid.

Where the carrier liquids are water miscible, examples of suitable water soluble materials include:-
- Water soluble vitamins such as ascorbic acid;
- water soluble fluorescers such as the 4,4'-bis(sulfostyryl)biphenyl disodium salt (sold under the trade name Tinopal CBS-X;
- activated aluminium chlorohydrate and other antiperspirant actives;
- transition metal complexes used as bleaching catalysts;
- water soluble polymers such as polyesters isophthalic acid), gerol, xanthan gum, or polyacrylates;
- diethylenetriaminepentaacetic acid (DTPA);
or mixtures thereof

Where the carrier material is hydrophobic, the dopant may be any of the materials described above as suitable for incorporation by solution in a hydrophilic carrier material. Preferred dopants include, for example, anti-perspirant actives (such as AACH) and metal complexing agents (such as DTPA). Similarly, the hydrophobic carrier may also contain, in dissolved form, one or more of the hydrophobic materials described above as hydrophobic dopants.

### Method of Preparation:

The intermediate emulsions are typically prepared under conditions which are well known to those skilled in the art, for example, by using a magnetic stirring bar, a homogenizer, or a rotator mechanical stirrer.

Cooling of the emulsion may be accomplished by introducing the emulsion into a fluid freezing medium, either directly, for example by pouring, dropping or spraying or in a mould. Preferably, the freezing medium is at a temperature below the freezing point of all components of the emulsion and is preferably at a much lower temperature to facilitate rapid freezing.

The freezing medium is preferably a liquified substance which is a gas or vapour at ambient temperature and pressure. The freezing medium may be at its boiling point during the freezing of the liquid medium or it may be cooled to below its boiling point by external cooling means.

The fluid freezing medium may be selected from one or more of the following group; liquid air, liquid nitrogen (b.p. -196°C), liquid ammonia (b.p. -33°C), liquified noble gas such as argon, liquefied halogenated hydrocarbon such as trichloroethylene, chlorofluorocarbons such as Freon(TM), hexane, dimethylbutene, isoheptane or cumene. Mixtures of organic liquids and solid carbon dioxide may also be used as the fluid freezing medium. Examples of suitable mixtures include chloroform or acetone and solid carbon dioxide (-77°C) and diethyl ether and solid carbon dioxide (-100°C).

Due to the very low boiling temperature, unreactivity, ease of expulsion and economy, liquid nitrogen is a preferred fluid freezing medium.

Water and the second liquid phase may be removed from the frozen emulsion by exposing the frozen liquid medium to high vacuum. The conditions for freeze drying will be well known to those skilled in the art and the vacuum to be applied and the time taken should be such that effectively all of the water and the second liquid phase is removed by sublimation. Preferably the materials removed during freeze-drying are captured for re-use.

Freeze-drying may take place with the frozen emulsion still in the mould. Alternatively, the frozen emulsion may be removed from the mould and subsequently freeze-dried. The freeze-drying step may be performed for up to around 72 hours.

In a preferred process according to the invention the emulsion has a relatively volatile continuous oil phase (containing a less volatile oil-soluble carrier material) and a discontinuous aqueous phase (containing a hydrophilic dopant). When this emulsion is freeze-dried and returned to ambient temperature, the product is a hydrophobic carrier liquid with a hydrophilic dopant.

Surfactants can be present as emulsifiers. Surfactants suitable for use as emulsifiers in oil-in-water emulsions preferably have an HLB value in the range 8 to 18. It is preferred that the surfactant is present in the liquid medium in a concentration of about 1% to about 60% by weight. More preferably, the surfactant is present in the liquid medium in a concentration of about 2 % to about 40 % by weight and a yet more preferred concentration is about 5% to about 25% by weight.

The method according to the present invention, will be more particularly described, by way of example only, with reference to the accompanying Examples.

### Examples:

The freeze-drier used in the examples was an Edwards Supermodulyo^{™}. This was operated with an average vacuum of 0.2mbar and at -50 °C.

### Example 1 - Hydrophobic Surfactant as the carrier, Hydrophilic (MACH) dopant:

In this example the alkoxydimethicone ABIL^{™} EM90 surfactant (ex Goldschmidt) was used as the carrier material. The dopant was the hydrophilic antiperspirant active known as activated aluminium chlorohydrate (AACH).

ABIL EM90 (2.0 g) as the carrier was dissolved in the volatile silicone DC24S^{™} ex Dow Corning (16 ml > 65 wt.% decamethylcyclopentasiloxane) to form a mixture which would become the continuous phase of the emulsion. The dispersed phase of the emulsion was prepared separately and comprised AACH (0.56 g) in water (48 ml). The two phases were combined by slowly adding the aqueous phase to the silicone with vigorous stirring using a type RW11 Basic IKA paddle stirrer.

The emulsion formed was frozen by placing its container in liquid nitrogen. The resulting frozen body was freeze-dried and subsequently allowed to return to ambient temperature to form a liquid. The resulting liquid is believed to consist of ABIL EM90 (a water-immiscible silicone) with AACH (a hydrophilic active) dispersed therein. This liquid dissolved readily into DC245 for form a slightly cloudy `solution'.

### Example 2 - Hydrophobic Surfactant as the carrier, hydrophilic (DTPA) dopant:

In this example the alkoxydimethicone ABIL^{™} EM90 surfactant (ex Goldschmidt) was used as the carrier material. The dopant was the hydrophilic complexing agent diethylenetriamine pentaacetic acid (DTPA).

ABIL EM90 (4.0 g) was dissolved in DC245 (96 ml) to form the continuous phase. To this organic solution was added the internal phase comprising DTPA (1.92 g) in water (94.1 ml, the pH was adjusted to 7 using potassium hydroxide) with vigorous stirring (as described in example 1). The emulsion formed was freeze-dried and on warming formed a liquid which dissolved readily into DC245 for form a clear 'solution'.

## Claims

1. A method for the preparation of a carrier liquid which comprises the steps of:
(I) preparing an emulsion comprising:
a) An aqueous phase,
b) A second liquid phase which is volatile and which is immiscible with aqueous phase (a),
c) A carrier material which is soluble in the continuous phase of the emulsion and which is liquid at ambient temperature, and
d) A dopant which is soluble in the disperse phase of the emulsion,
(II) cooling the emulsion produced in step (I) to a temperature at which at least both the continuous phase and the carrier material become solid,
(III) removing water and the volatile second phase from the cooled emulsion in vapour form, and,
(IV) returning the product of step (III) to ambient temperature to obtain a liquid product with the dopant dispersed therein.

2. A method according to claim 1 wherein the carrier material is soluble in the second liquid phase and said second liquid phase forms the continuous phase of the emulsion formed in step (I).

3. A method according to claim 2 wherein the second liquid phase comprises one or more materials selected from selected from: alkanes, cyclic hydrocarbons, halogenated alkanes, esters, ketones, ethers and, silicones.

4. A method according to claim 3 wherein the carrier material comprises one or more materials selected from natural, mineral or synthetic oils less volatile than the second liquid phase.

5. A method according to claim 3 wherein the second liquid phase comprises a volatile cyclic silicone.

6. A method according to claim 4 wherein the carrier material comprises an alkoxy dimethicone.

7. A method according to claim 1 wherein the dopant is hydrophilic and is selected from on or more of: water soluble vitamins; water soluble fluorescers; water soluble antiperspirant actives; transition metal complexes having activity as bleaching catalysts; and, water soluble polymers and water soluble metal chelating agents.

8. A method according to claim 7 wherein the dopant is selected from diethylene-triamine-penta-acetic acid, activated aluminium chlorohydrate and mixtures thereof.

9. A method according to claim 1 wherein step (II) comprises introducing the emulsion into a fluid freezing medium.

10. A method according to claim 1 wherein step (III) comprises exposure to vacuum.

## Patentansprüche

1. Verfahren zur Herstellung einer Trägerflüssigkeit, das die folgenden Schritte umfasst:
(I) Herstellen einer Emulsion, umfassend:
a) eine wässrige Phase,
b) eine zweite flüssige Phase, die flüchtig ist und die mit der wässrigen Phase (a) nicht mischbar ist,
c) ein Trägermaterial, das in der kontinuierlichen Phase der Emulsion löslich ist und das bei Umgebungstemperatur flüssig ist, und
d) ein Dotierungsmittel, das in der dispersen Phase der Emulsion löslich ist,
(II) Kühlen der in Schritt (I) hergestellten Emulsion auf eine Temperatur, bei der wenigstens beide, die kontinuierliche Phase und das Trägermaterial, fest werden,
(III) Entfernen von Wasser und der flüchtigen zweiten Phase aus der gekühlten Emulsion in Dampfform und
(IV) Zurückbringen des Produkts von Schritt (III) auf Umgebungstemperatur, um ein flüssiges Produkt mit dem Dotierungsmittel darin dispergiert zu erhalten.

2. Verfahren nach Anspruch 1, wobei das Trägermaterial in der zweiten flüssigen Phase löslich ist und die zweite flüssige Phase die kontinuierliche Phase der in Schritt (I) gebildeten Emulsion bildet.

3. Verfahren nach Anspruch 2, wobei die zweite flüssige Phase ein Material oder mehrere Materialien umfasst, ausgewählt aus: Alkanen, cyclischen Kohlenwasserstoffen, halogenierten Alkanen, Estern, Ketonen, Ethern und Silikonen.

4. Verfahren nach Anspruch 3, wobei das Trägermaterial ein Material oder mehrere Materialien, ausgewählt aus natürlichen, mineralischen oder synthetischen Ölen, die weniger flüchtig sind als die zweite flüssige Phase, umfasst.

5. Verfahren nach Anspruch 3, wobei die zweite flüssige Phase ein flüchtiges cyclisches Silikon umfasst.

6. Verfahren nach Anspruch 4, wobei das Trägermaterial ein Alkoxydimethicon umfasst.

7. Verfahren nach Anspruch 1, wobei das Dotierungsmittel hydrophil ist und ausgewählt ist aus einem oder mehreren von: Wasser, löslichen Vitaminen; wasserlöslichen Fluoreszenzmitteln; wasserlöslichen Antiperspirant-Wirkstoffen; Übergangsmetallkomplexen, die Aktivität als Bleichkatalysatoren haben, und wasserlöslichen Polymeren und wasserlöslichen Metallchelatbildnern.

8. Verfahren nach Anspruch 7, wobei das Dotierungsmittel aus Diethylentriaminpentaessigsäure, aktiviertem Aluminiumchlorhydrat und Gemischen davon ausgewählt wird.

9. Verfahren nach Anspruch 1, wobei Schritt (II) Einführen der Emulsion in ein Fluid-Gefriermedium umfasst.

10. Verfahren nach Anspruch 1, wobei Schritt (III) eine Exposition gegenüber Vakuum umfasst.

## Revendications

1. Méthode de préparation d'un liquide support qui comprend les étapes consistant à :
(I) préparer une émulsion comprenait
(a) une phase aqueuse ;
(b) une seconde phase liquide qui est volatile et qui est immiscible avec la phase aqueuse (a),
(c) un matériau de support qui est soluble dans la phase continue de l'émulsion et qui est liquide à la température ambiante, et
(d) un agent dopant qui est soluble dans la phase dispersée de l'émulsion,
(II) refroidir l'émulsion produite dans l'étape (I) à une température à laquelle au moins la phase continue et le matériau de support deviennent solides,
(III) enlever l'eau et la seconde phase volatile de l'émulsion refroidie sous forme de vapeur, et
(IV) reporter le produit de l'étape (III) à la température ambiante pour obtenir un produit liquide avec l'agent dopant dispersé à l'intérieur.

2. Méthode selon la revendication 1, dans laquelle le matériau de support est soluble dans la seconde phase liquide et ladite seconde phase liquide forme la phase continue de l'émulsion formée dans l'étape (I).

3. Méthode selon la revendication 2, dans laquelle la seconde phase liquide comprend un ou plusieurs matériaux choisis parmi : des alcanes, des hydrocarbures cycliques, des alcanes halogénés, des esters, des cétones, des éthers et des silicones.

4. Méthode selon la revendication 3, dans laquelle le matériau de support comprend un ou plusieurs matériaux choisis parmi des huiles naturelles, minérales ou synthétiques moins volatiles que la seconde phase liquide.

5. Méthode selon la revendication 3, dans laquelle la seconde phase liquide comprend un silicone cyclique volatile.

6. Méthode selon la revendication 4, dans laquelle le matériau de support comprend un alcoxy-diméthicone.

7. Méthode selon la revendication 1, dans laquelle l'agent dopant est hydrophile et est choisi parmi un ou plusieurs des éléments parmi : les vitamines solubles dans l'eau, les agents fluorescents solubles dans l'eau, les actifs anti-transpirants solubles dans l'eau ; les complexes de métal de transition ayant une activité en tant que catalyseurs de blanchiment, et des polymères solubles dans l'eau et des agents chélatants métalliques solubles dans l'eau.

8. Méthode selon la revendication 7, dans laquelle l'agent dopant est choisi parmi un acide diéthylène-triamine-penta-acétique, un chlorhydrate d'aluminium activé et des mélanges correspondants.

9. Méthode selon la revendication 1, dans laquelle l'étape (III) comprend l'introduction de l'émulsion dans un milieu de congélation fluide.

10. Méthode selon la revendication 1, dans laquelle l'étape (III) comprend l'exposition au vide.
